(19) Europäisches Patentamt
European Patent Office
Office européen des brevets

(11) Veröffentlichungsnummer: **0 122 576**
**B1**

(12) # EUROPÄISCHE PATENTSCHRIFT

(45) Veröffentlichungstag der Patentschrift: **06.11.85**

(21) Anmeldenummer: **84103945.6**

(22) Anmeldetag: **09.04.84**

(51) Int. Cl.⁴: **C 07 C 45/27**, C 07 C 51/16, C 07 C 67/16, C 07 C 67/30, C 07 C 102/00, C 07 C 49/12, C 07 C 69/66, C 07 C 69/36, C 07 C 103/34

(54) **Verfahren zur Herstellung von vicinalen Polycarbonylverbindungen.**

(30) Priorität: **16.04.83 DE 3313917**

(43) Veröffentlichungstag der Anmeldung: **24.10.84 Patentblatt 84/43**

(45) Bekanntmachung des Hinweises auf die Patenterteilung: **06.11.85 Patentblatt 85/45**

(84) Benannte Vertragsstaaten: **BE CH DE FR GB IT LI NL**

(56) Entgegenhaltungen:
**DE - B - 1 226 562**
**DE - C - 573 722**

**Patent Abstracts of Japan, unexamined applications, Sektion C, Band 1,Nr. 131, 28. Oktober 1977**
**Chemische Berichte, 115. Jahrgang, Heft 9, September 1982, Weinheim. K. Schank et al. "Ozonspaltung von Sulfonium-Yliden" Seiten 3032-3041**

(73) Patentinhaber: **BASF Aktiengesellschaft, Carl-Bosch-Strasse 38, D-6700 Ludwigshafen (DE)**

(72) Erfinder: **Koenig, Horst, Prof. Dr., Pariser Strasse 4, D-6700 Ludwigshafen (DE)**
Erfinder: **Eisen, Gerhard, Schillerstrasse 5, D-6707 Schifferstadt (DE)**

**EP 0 122 576 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Diese Erfindung betrifft ein Verfahren zur Herstellung von vicinalen Polycarbonylverbindungen durch Photooxidation von Schwefelyliden.

Vicinale Polycarbonylverbindungen, wie 1,2-Di- und 1,2,3-Tricarbonylverbindungen, sind wichtige Ausgangsstoffe für die Herstellung heterocyclischer Verbindungen, die z.B. als Wirkstoffe für Arzneimittel oder Pflanzenschutzmittel oder als Zwischenprodukte für die Herstellung von Farbstoffen verwendet werden (s. u.a. „Chemical Reviews", 1975, Vol. 75, No. 2, S. 177 bis 202 und DE-OS Nr. 2710902, S. 25 bis 27).

Nach den bisher bekannten Methoden lassen sich vicinale Polycarbonylverbindungen durch eine Oxidation der Methylengruppen der entsprechenden β-Dicarbonylverbindungen z.B. durch Behandlung mit Selendioxid („Helv. Chimica Acta" *57*, Fasc. 7 (1974), S. 2201 bis 2208) mit Nitrosoverbindungen oder mit Diazoniumsalzen („Chemical Reviews", 1975, Vol. 75, No. 2, S. 178 und 184) herstellen. Diese Verfahren haben den grossen Nachteil des Einsatzes toxischer oder explosiver Substanzen. Nach den Angaben in „Chem. Ber." *115* (1982), S. 3032 bis 3041, kann man Schwefelylide, die bekanntlich von üblichen Oxidationsmitteln nicht angegriffen werden, durch Behandlung mit Ozon in die entsprechenden Keto-Verbindungen überführen. Diese Methode hat den Nachteil eines hohen Energieverbrauchs und des Bedarfs spezieller Apparaturen.

Man hat auch schon versucht, Schwefelylide durch Photooxidation mit Methylenblau und Tetraphenylporphin als Sensibilisatoren in die gewünschten Keto-Verbindungen zu überführen. Dieses in „Photochemistry and Photobiology" *30* (1979), S. 81 bis 87 beschriebene Verfahren ist nicht auf alle Ylide anwendbar. So erhält man z.B. aus Bisacetyldimethylsulfonium-methylid lediglich Essigsäure und aus Phenacyldimethyl-sulfoniumylid nur Benzoesäure und Benzoesäuremethylester. Die Photooxidation mit Methylenblau hat auch den Nachteil längerer Reaktionszeiten, so dass ausbeutemindernde Folge- und Nebenreaktionen in den Vordergrund treten. Ausserdem wird mehr Energie benötigt und eine wesentlich schlechtere Raum-Zeit-Ausbeute erreicht.

Da Schwefelylide leicht zugängliche Ausgangsstoffe darstellen, war nach einem Verfahren zu suchen, das es ermöglicht, diese Ausgangsstoffe nach einer allgemein anwendbaren vorteilhaften Methode in die gesuchten vicinalen Polycarbonylverbindungen zu überführen.

Diese Aufgabe wurde durch das erfindungsgemässe Verfahren gelöst. Nach dem Verfahren der Erfindung kann man vicinale Polycarbonylverbindungen der Formel

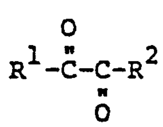 (I)

in der $R^1$ einen Alkyl- oder Arylrest, den $H_5C_2O$-Rest, oder einen $R^5R^6N$-Rest, in dem $R^5$ für einen Alkyl- oder Arylrest und $R^6$ für ein Wasserstoffatom oder einen Alkylrest steht und $R^2$ ein Wasserstoffatom, einen Alkylrest oder einen $R^7OC$- oder $R^7OOC$-Rest, in denen $R^7$ für einen Alkylrest steht, bedeuten, dadurch herstellen, dass man Schwefelylide der Formel

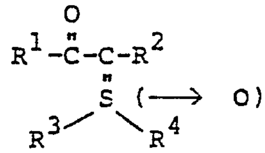 (II)

in der die Reste $R^3$ und $R^4$ jeweils Alkyl- oder Arylreste oder beide Reste $R^3$ und $R^4$ zusammen einen Alkylenrest bedeuten, photochemisch mit Bengalrosa (4,5,6,7-Tetrachlor-2',4',5',7'-tetraiodfluorescein) als Sensibilisatoren oxidiert.

Das Verfahren der Erfindung lässt sich formelmässig so beschreiben:

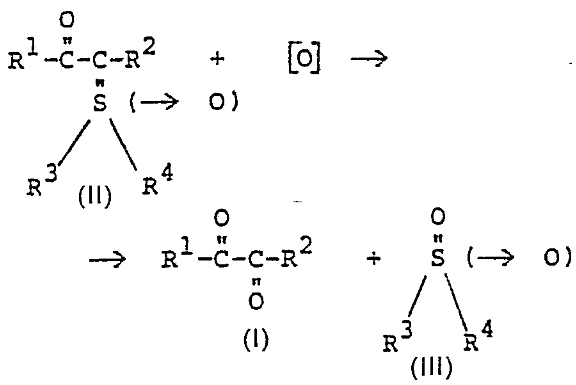

Die Schwefelylide der Formel II sind z.B. nach den in den deutschen Patentschriften Nrn. 1207379 und 1226561 beschriebenen Verfahren erhältlich.

In den Schwefelyliden der Formel II bedeuten Alkylreste z.B. Alkylreste mit 1 bis 12, vorzugsweise 1 bis 4 C-Atomen. Arylreste sind z.B. Phenylreste, die z.B. noch durch niedere Alkylreste, wie Methyl- oder Ethylgruppen oder durch Halogenatome substituiert sein können.

Man photooxidiert die Ylide in Gegenwart von Bengalrosa z.B. bei Temperaturen bis 100°C, vorzugsweise bei −20 bis +40°C. Als Lösungsmittel sind z.B. Wasser, niedermolekulare Alkohole, wie Methanol und Ethanol, niedermolekulare Säuren, wie Essigsäure, Chlorkohlenwasserstoffe, wie Methylenchlorid, Aromaten, wie Benzol, Chlorbenzol oder Toluol, Ether, niedermolekulare Ketone, wie Aceton oder Mischungen dieser Lösungsmittel geeignet. Den Sensibilisator gibt man z.B. in Methanol oder Ethanol gelöst bzw. der Reaktionslösung in fester Form zu.

Das Reaktionsende lässt sich leicht durch dünnschichtchromatographische Analyse des unverbrauchten Ylids bestimmen. Von der Sulfoxid- und Sulfonverbindung der Formel III lässt sich die gewünschte Polycarbonylverbindung leicht durch Destillation, Kristallisation oder Extraktion bzw. durch direkte Weiterreaktion zum gewünschten Endprodukt abtrennen. Hydrate, Acetale und Perhydroxy-addukte der Reaktionsprodukte werden gegebenenfalls durch bekannte Methoden gespalten.

Nach dem neuen Verfahren erhält man die vicinalen Polycarbonylverbindungen glatt und in guter Ausbeute. Dieses vorteilhafte Ergebnis war nicht zu erwarten. So ist nämlich im Fortschritte der „Chem. Forschung" Band *9* (1968), S. 506 angegeben, dass sich die Schwefelylide der Formel II mit Wasserstoffperoxid nicht oxidieren lassen.

*Beispiel 1:*

In einem 1-l-Photoreaktor, der einen 150 Watt Hg-Hochdruckbrenner in einem wassergekühlten Pyrex-Tauchschacht enthält, werden 9,7 g (44 mmol) N,N-Dimethylcarbamoyl-äthoxycarbonyl-dimethylsulfoniumylid und 0,2 g Bengalrosa in 600 ml Ethanol gelöst. Die tiefrote Lösung wird bei 12°C gerührt und nach Einschalten des Brenners mit 20 l/h Sauerstoff begast. Nach 1 h ist kein Ausgangsylid mehr nachweisbar (DC). Der hellrote, homogene Reaktorinhalt wird eingeengt (Rotationsverdampfer, 45°C Badtemperatur, 20 Torr). Der Rückstand wird im Kugelrohr destilliert. Man erhält 5,86 g (33,8 mmol), 77% d.Th.) N,N-Dimethyl-carbamoyloxalsäure-ethylester.

H–NMR (80 MHz, $CDCl_3$), t 1,30 (3H, $-OCH_3CH_3$), s 3,10 (6H, $NCH_3$), q 4,40 (2H, $-OCH_2-CH_3$).

*Beispiel 2:*

Man gibt in den in Beispiel 1 beschriebenen Photoreaktor 400 ml Schwefelkohlenstoff und 150 ml Ethanol. Es wird auf 10°C gekühlt und mit 50 l $O_2$/h unter Rühren begast Innerhalb von 15 Minuten wird die Lösung von 5,4 g (20 mmol) N-Phenylcarbamoyl-ethoxycarbonyl-dimethylsulfoniummethylid und 0,5 g Bengalrosa in 110 ml Ethanol zugegeben. Da der Farbstoff verblasst, werden alle 30 Minuten weitere 0,1 g Bengalrosa zugegeben. Nach 4 Stunden ist alles Ylid oxidiert. Der Schwefelkohlenstoff wird bei 50°C Badtemperatur abdestilliert, zuletzt unter Zusatz von Methylenchlorid. Man engt dann im Vakuum ein und erhält 8,8 g rotes Öl, das nach der Dünnschichtchromatographie zu 80 bis 90% aus dem Mesoxalsäuremonoethylesteranilid besteht.

Die Gehaltsbestimmung erfolgt über das 2,4-Dinitrophenylhydrazon (3,53 g, 16 mmol, 80% d.Th.) des Mesoxalsäuremonoethylesteranilids (Schmp. 208 bis 210°C, $CH_3OH/CH_2Cl_2$).

H–NMR (80 MHz, $CDCl_3$): t 1,47 ppm (3H, $-OCH_2-CH_3$), q 4,45 ppm (2H, $-OCH_2-CH_3$), m 7,08-8,6 ppm (8H, aromatische Protonen), 11,0 ppm und 15,8 ppm (je 1 NH).

*Analyse* für $C_{17}H_{15}N_5O_7$ (MG 401,33)

| | | | | |
|---|---|---|---|---|
| Ber.: | C 50,88 | H 3,77 | N 17,45 | O 27,91% |
| Gef.: | C 50,4 | H 3,6 | N 17,9 | O 28,1 % |

*Beispiel 3:*

In einem 200 ml Photoreaktor, der einen 125 Watt Hg-Hochdruckbrenner (Philipps 125 HPK) enthält, werden 3,8 g (20 mmol) Acetylethoxycarbonyl-dimethylsulfonium-methylid in 120 ml Schwefelkohlenstoff und 15 ml Methanol gelöst und unter Zusatz von 0,3 g Bengalrosa 1 ½ h mit 50 l $O_2$/h begast. Der dabei entstandene Acetyloxalethylester wird in situ mit 20 mmol Benzamidrazon (gelöst in 50 ml Ethanol) umgesetzt. Nach 17 h bei 23°C engt man ein und gewinnt durch Destillation bei 150°C/0,4 Torr und anschliessende Kristallisation aus Ligroin (20 ml) 1,1 g (52% d.Th.) 3-Phenyl-5-methyl-6-ethoxycarbonyl-1,2,4-triazin mit Schmp. 72-73°C.

H–NMR (80 MHz, $CDCl_3$): t 1,5 ppm (3H, $-OCH_2CH_3$), s 2,85 ppm (3H, $CH_3$-Triazin), q 4,60 ppm (2H, $-OCH_2CH_3$), m 7,6 ppm (3H, Phenyl), m 8,6 ppm (2H, Phenyl).

*Analyse* für $C_{13}H_{13}N_3O_2$ (MG 243,26)

| | | | | |
|---|---|---|---|---|
| Ber.: | C 64,19 | H 5,39 | N 17,27 | O 13,15% |
| Gef.: | C 64,2 | H 5,4 | N 17,2 | O 13,2 % |

*Beispiel 4:*

97,4 g (450 mmol) Acetylethoxycarbonyl-tetramethylen-sulfoniummethylid und 0,9 g Bengalrosa werden in 150 ml Ethanol und 850 ml Schwefelkohlenstoff gelöst. Man kühlt auf −15°C und begast im Photoreaktor (s. Beispiel 1) mit 50 l $O_2$/h.

Nach 9 h 45 min ist das Ylid völlig verbraucht (DC-Kontrolle). Der Schwefelkohlenstoff wird sorgfältig abdestilliert, dann werden bei 80°C/ 5 Torr alle flüchtigen Bestandteile abgezogen. Man erhält 52,2 g des rohen Acetyloxalethylesters als braunes Öl. Die Gehaltsbestimmung nimmt man über das Bis-2,4-Dinitrophenylhydrazon vor. Die Ausbeute beträgt 73,8% d.Th.

*Beispiel 5:*

Man gibt 100 ml Schwefelkohlenstoff und 20 ml Diethylether in den in Beispiel 3 beschriebenen Photoreaktor und bestrahlt unter einem Sauerstoffstrom von 40 l/h. Nach 5 min beginnt man die Lösung von 4,4 g (20 mmol) Diethoxycarbonyldimethylsulfonium-methylid in 20 ml Methanol und 100 mg Bengalrosa zuzugeben. Der ausbleichende Farbstoff wird durch insgesamt 400 mg frisches Bengalrosa portionsweise während der Reaktionsdauer von 150 min ersetzt. 30 min nach Ende der Ylidzugabe ist dieses völlig umgesetzt und die Reaktion wird gestoppt. Durch Abdestillieren der Lösungsmittel erhält man 6,1 g rotes Öl mit festen Anteilen. Man destilliert im Kugelrohr bei 120 bis 125°C/14 mbar und erhält 81,6% d.Th. an Mesoxalsäureethylester (bestimmt als 2,4-Dinitrophenylhydrazon, Schmp. 116 bis 117°C).

*Beispiel 6:*

Der in Beispiel 1 beschriebene Photoreaktor wird mit einer Lösung von 10,3 g (50 mmol) Benzoyl-tetramethylensulfonium-methylid und 0,3 g Bengalrosa in 750 ml Methanol beschickt. Bei 12°C und unter Rühren werden in 3 h 150 l Sauerstoff über eine Fritte durch die tiefrote Reaktionslösung geleitet. Durch Dünnschichtchromatographie (System $SiO_2$, n-Butanol, Wasser, Essigsäure = 4:1:1) lässt sich kein Ausgangsylid (Rf 0,17)

nachweisen. Als Reaktionsprodukte werden Phenylglyoxylsäure (Rf 0,60) und deren Methylester (Rf 0,95) festgestellt. Als Hauptprodukt beobachtet man im System $SiO_2$/Methylenchlorid + 5% Isopropanol/Phenylglyoxal (Rf 0,59). Der gesamte Reaktionsansatz wird filtriert und das rotgefärbte Filtrat mit 5,4 g (50 mmol) o-Phenylendiamin versetzt. Unter rascher Verfärbung nach Dunkelbraunrot reagiert das Phenylglyoxal zu 2-Phenylchinoxalin (Rf 0,68, $SiO_2$/Methylenchlorid + 5% Isopropanol).

Man engt bei 40° C unter Vakuum ein und reinigt das erstarrende braune Öl über 400 g Kieselgel 60 mit Methylenchlorid als Laufmittel. Man isoliert ein gelbes Kristallisat, das nach einmaligem Umlösen aus Methanol bei 77 bis 78° C schmilzt. Ausbeute 5,6 g 2-Phenylchinoxalin, das sind 54,3% d.Th. bezogen auf eingesetztes Ylid.

*Beispiel 7:*

Man gibt in den in Beispiel 1 beschriebenen Photoreaktor 750 ml Methanol und 0,2 g Bengalrosa und leitet 5 Minuten unter Belichtung Sauerstoff ein. Dann werden 0,3 g Bengalrosa, 13,1 g (50 mmol) Phenacyldimethylsulfoniumbromid in 150 ml Methanol und 50 ml 1 n Natronlauge zugetropft (Zulaufgeschwindigkeit 80 $ml.h^{-1}$). Die Reaktorinnentemperatur erreicht durch Eiskühlung 12 bis 15° C. Der Reaktionsverlauf wird dünnschichtchromatographisch verfolgt. Der Test auf Ausgangsylid (Rf 0,15) und die gebildete Phenylglyoxylsäure (Rf 0,60) erfolgt im System $SiO_2$, n-Butanol, Wasser, Essigsäure = 4:1:1. Der Test auf Phenylglyoxal (Rf 0,59), Phenylglyoxylsäuremethylester (Rf 0,80) und Benzoesäure (Rf 0,50) erfolgt im System $SiO_2$, Methylenchlorid + 5% Isopropanol.

Nach 2 ¾ h Zulaufzeit und ½ h Nachreaktion ist das Ausgangsmaterial vollständig umgesetzt.

Die DC-Kontrollen zeigen als Hauptprodukt den Ester neben wenig Säure und geringen Mengen Phenylglyoxal und Benzoesäure.

Die homogene, hellrote Lösung wird am Rotationsverdampfer bei 40° C eingeengt, bis Methanol weitgehend entfernt ist. Aus dem Restvolumen von ca. 80 ml scheidet sich ein Öl ab. Im Scheidetrichter wird mit Methylenchlorid extrahiert und über Natriumsulfat getrocknet. Nach Einengen im Vakuum bei 40° C erhält man 9,1 g braunrotes Öl. Zur Rückstandstrennung wird im Kugelrohr destilliert. Man erhält 8,1 g braunes Öl, $KP_{0,25}$ 70 bis 110° C Manteltemperatur. Nach Feindestillation erhält man 4,7 g (28,6 mmol) Phenylglyoxylsäuremethylester (57,2% d.Th.) als farbloses Öl mit Siedepunkt 77 bis 80° C/0,3 mbar.

*Beispiel 8:*

14,4 g (50 mmol) Phenacyl-tetramethylensulfonium-bromid werden in 250 ml Wasser gelöst und mit 60 ml 2n Natronlauge versetzt. Im Photoreaktor (s. Beispiel 1) mischt man dazu 500 ml Methanol und 0,3 g Bengalrosa. Man leitet während der Bestrahlung Sauerstoff durch die Lösung. Nach 1 ½ h Bestrahlung bei 15°C sind 75 l $O_2$ durchgeleitet und das Ausgangsmaterial vollständig umgesetzt. Die DC-Kontrolle erfolgt im System $SiO_2$, n-Butanol, Wasser, Essigsäure = 4:1:1. Ylid-Rf 0,17; Phenylglyoxylsäure-Rf 0,60 ist als Hauptprodukt entstanden.

Der Reaktorinhalt wird bei 40° C am Rotationsverdampfer von Methanol befreit. Die verbleibende alkalische, wässerige Lösung wird mit Methylenchlorid gewaschen. Die Wasserphase wird am Rotationsverdampfer vom restlichen Lösungsmittel getrennt. Die rosafarbene Lösung wird bei Eiskühlung mit Salzsäure auf pH 1 bis 2 gestellt. Dabei fällt eine rosafarbene Festsubstanz aus, die aus Bengalrosa und Benzoesäure besteht.

Zum gerührten sauren Filtrat werden 5,4 g (50 mmol) Phenylhydrazin getropft, dabei fällt ein gelbbrauner Festkörper aus. Er wird nach 2 h abgesaugt, mit Wasser gewaschen und aus Methanol/Wasser umkristallisiert. Nach Trocknen im Vakuum erhält man 6,1 g (25,4 mmol = 50,8% d.Th.) Phenylglyoxylsäure-phenylhydrazon, Fp. 166 bis 167° C.

*Beispiel 9:*

In dem in Beispiel 1 beschriebenen Photoreaktor werden 4,0 g (25 mmol) Bisacetyl-dimethylsulfonium-methylid und 0,8 g Bengalrosa in 100 ml Ethanol gelöst und mit 700 ml Schwefelkohlenstoff gemischt. Der tiefrote Reaktorinhalt wird durch Aussenkühlung auf 4°C gekühlt. Die Photooxidation wird bei 4 bis 7° C im Sauerstoffstrom von 50 $l.h^{-1}$ durchgeführt. Die dünnschichtchromatographische Reaktionskontrolle im System $SiO_2$, Methylenchlorid + 5% Isopropanol zeigt den Umsatz des Ausgangsmaterials (Rf 0,10) an. Das entstehende Triketon wird durch Besprühen der DC-Platte mit 2,4-Dinitrophenylhydrazin-Lösung als orangefarbener Fleck mit Rf 0,32 nachgewiesen. Als Nebenprodukt wird Acetaldehyd angezeigt. Das während der Photooxidation verblassende Bengalrosa wird durch Zugabe von mehreren 0,1 g-Portionen des Farbstoffes ersetzt. Nach 4 Stunden ist das Ausgangsylid vollständig umgesetzt. Die blassrote Lösung wird durch Destillation am Rotationsverdampfer bei Normaldruck und 45°C Badtemperatur von der Hauptmenge des Schwefelkohlenstoffs befreit. Zum Rückstand werden 100 ml Methylenchlorid gegeben. Dann wird erneut abgedampft um den Schwefelkohlenstoff vollständig zu entfernen. Das restliche Lösungsmittel und leicht flüchtige Anteile werden dann bis zu einem Enddruck von 50 mbar abgezogen. Es werden als Eindampfrückstand 29 g des gebildeten 2,3,4-Triketopentans in Form einer braunroten Lösung erhalten. Zur Isolierung wird das 2,3,4-Triketopentan in das bis-2,4-Dinitrophenylhydrazon überführt. Dazu wird der Eindampfrückstand mit 400 ml 2,4-Dinitrophenylhydrazin-Lösung (Gehalt 0,13 $mmol.ml^{-1}$) bei Raumtemperatur über Nacht gerührt. Die gebildeten Festkörper werden abgesaugt und gut mit Wasser gewaschen. Der braunrote Rückstand wird in etwa 0,5 l Methylenchlorid gerührt. Die Suspension wird mit 200 ml 1 n Natronlauge intensiv geschüttelt. Das Bengalrosa geht dabei als Na-Salz in Lösung. Nach der Phasentrennung im

Scheidetrichter wird die Methylenchloridphase dreimal mit 1 n Natronlauge und einmal mit Wasser gewaschen. Die organische Phase wird über Natriumsulfat getrocknet und nach der Filtration am Rotationsverdampfer auf ca. 200 ml eingeengt. Zur Reinigung des Produktes wird die Lösung über 350 g Kieselgel, 0,04 bis 0,2 mm, mit Methylenchlorid als Laufmittel chromatographiert. Die Eluate werden dünnschichtchromatographisch getestet, System $SiO_2$, Methylenchlorid. Die Fraktionen, welche die Komponente mit dem DC-Rf 0,30 in reiner Form enthalten, werden vereinigt, am Rotationsverdampfer bei 40°C im Vakuum eingeengt und aus Ethanol umkristallisiert.

Man erhält 4,1 g (8,6 mmol) 2,3,4-Triketopentan-bis-2',4'-dinitrophenylhydrazon (34,5% d.Th.), Fp. 195°C Zers.

*Analyse* für $C_{17}H_{14}N_8O_9$ (MG 474,35)

| | | | | |
|---|---|---|---|---|
| Ber.: | C 43,05 | H 2,97 | N 23,62 | O 30,36% |
| Gef.: | C 43,2 | H 3,0 | N 23,6 | O 30,2 % |

*Beispiel 10:*

*Vergleichsversuche*

a) Methylenblau als Photosensibilisator

In dem im Beispiel 1 beschriebenen Photoreaktor werden 22,0 g (100 mmol) Diethoxycarbonyldimethylsulfoniummethylid in 120 ml Ethanol und 750 ml Schwefelkohlenstoff gelöst und mit 2,14 mmol (0,8 g) Methylenblau (Merck 6040) versetzt. Bei 12 bis 15°C werden 40 l/Std Sauerstoff unter Bestrahlung eingegast (Tauchfritte). Durch Dünnschichtchromatographie (System Mack 5719 ($SiO_2$), Methylenchlorid + 5% Isopropanol) wird die Umwandlung des Ylids kontrolliert. Nach 8 Stunden sind 50%, nach 15 Stunden 100% umgesetzt. Man destilliert die Lösungsmittel bei 50°C zuletzt am Rotationsverdampfer ab und erhält 31,4 g eines dunkelblauen Öls, das über eine kurze Brücke, bei 14 Torr destilliert wird. Man erhält 19,7 g hellblaues Öl im Intervall von 95 bis 102°C. Die Gehaltsbestimmung erfolgt durch Fällung des 2,4-Dinitrophenylhydrazons des gebildeten Mesoxalesters.

Man erhält 68,3% d.Th. in Form gelber Kristalle mit dem Schmelzpunkt 110 bis 111°C.

b) Bengalrosa als Photosensibilisator

Man verfährt wie im Absatz a) beschrieben, wobei man jedoch anstelle des Methylenblaus 1,6 mmol (1,6 g) Bengalrosa verwendet. Bereits nach 4½ Stunden ist das Ausgangsylid vollständig umgesetzt und man bestimmt den Mesoxalester in 76%iger Ausbeute durch Fällung des 2,4-Dinitrophenylhydrazons (Schmelzpunkt 114 bis 115°C).

**Patentanspruch**

Verfahren zur Herstellung von vicinalen Polycarbonylverbindungen der Formel

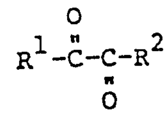

(I)

in der $R^1$ einen Alkyl- oder Arylrest, den $H_5C_2O$-Rest, oder einen $R^5R^6N$-Rest, in dem $R^5$ für einen Alkyl- oder Arylrest und $R^6$ für ein Wasserstoffatom oder einen Alkylrest steht und $R^2$ ein Wasserstoffatom, einen Alkylrest oder einen $R^7OC$- oder $R^7OOC$-Rest, in denen $R^7$ für einen Alkylrest steht, bedeuten, dadurch gekennzeichnet, dass man Schwefelylide der Formel

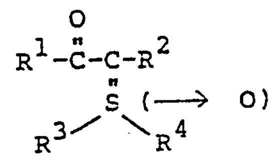

(II)

in der die Reste $R^3$ und $R^4$ jeweils Alkyl- oder Arylreste oder beide Reste $R^3$ und $R^4$ zusammen einen Alkylenrest bedeuten, photochemisch mit Bengalrosa als Sensibilisator oxidiert.

**Claim**

A process for the preparation of a vicinal polycarbonyl compound of the formula

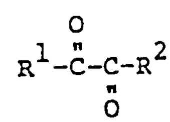

(I)

where $R^1$ is alkyl or aryl, $H_5C_2O$, or an $R^5R^6N$ radical, where $R^5$ is alkyl or aryl and $R^6$ is hydrogen or alkyl, and $R^2$ is hydrogen, alkyl, or an $R^7OC$ or $R^7OOC$ radical, where $R^7$ is alkyl, wherein a sulphur ylid of the formula

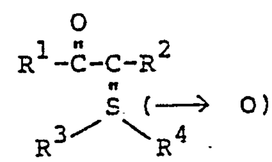

(II)

where $R^3$ and $R^4$ are each alkyl or aryl, or $R^3$ and $R^4$ together are an alkylene radical, is oxidized photochemically using rose bengal as sensitizer.

**Revendication**

Procédé pour la préparation de composés polycarbonyliques vicinaux de formule

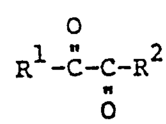

(I)

dans laquelle $R^1$ représente un radical alkyle ou aryle, le radical $H_5C_2O$ ou un radical $R^5R^6N$ dans lequel $R^5$ est mis pour un radical alkyle ou aryle et $R^6$ pour un atome d'hydrogène ou un radical alkyle, et $R^2$ représente un atome d'hydrogène, un radical alkyle ou un radical $R^7OC$ ou $R^7OOC$ dans lequel $R^7$ est mis pour un radical alkyle, caractérisé en ce qu'on oxyde photochimiquement, en utilisant le rose Bengale comme sensibilisateur, des ylures du soufre de formule

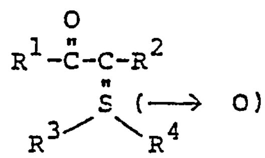

(II)

dans laquelle les radicaux $R^3$ et $R^4$ représentent chacun un radical alkyle ou aryle ou les deux radicaux $R^3$ et $R^4$ forment ensemble un radical alkylène.